# EUROPEAN PATENT APPLICATION

(11) **EP 1 125 591 A1**
(43) Date of publication of application: **22.08.2001**
(21) Application number: 00311556.5
(22) Date of filing: 21.12.2000
(51) Int. Cl.: A61M 1/34, G01N 33/49

(54) **Instrument and method for blood separation**

(30) Priority: 05.01.2000 JP 2000000297
(71) Applicant: Maehata, Eisuke, Hachioji-Shi, Tokyo (JP)
(72) Inventor: Nanba, Hiromi, Nakano-ku, Tokyo (JP); Koga, Osamu, Minamitakaki-Gun, Nagasaki-Ken (JP)
(74) Representative: Skone James, Robert Edmund

(57) **Abstract**

An object of the present invention is to reduce the cost in blood test and to simplify the operation thereof.

According to the present invention, there is provided an instrument to be used for blood separation, comprising: a blood collection means 8 for containing collected blood 38; a filtration means 3 for separating blood cell 40 and blood plasma 39 in said collected blood; a blood cell collection means 5 for containing separated blood cell; a blood plasma collection means 4 for containing separated blood plasma; and a pressure applying means 6 for applying a pressure to said blood contained in said blood collection means; wherein said filtration means has a capillary 12 in communication with a blood discharging section of said blood collection means and a blood cell introducing section of said blood cell collection means, said capillary 12 having a plurality of perforations each being formed through a wall thereof; said instrument being characterized in that: said blood in said blood collection means is introduced into said capillary by a pressure applying motion caused by said pressure applying means; and the blood plasma in said blood moves through said perforation of said capillary to be separately contained in said blood plasma collection means, while the blood cell in said blood flows through said capillary to be contained in said blood cell collection container.

## Description

The present invention relates to an instrument and a method for blood separation, and in particular, an instrument and a method for separating collected blood into blood cell and blood plasma, right on the spot of collection.

Generally, a blood collection is categorized into a normal blood collection where a specifically qualified person, such as a doctor, collects blood from a vein by using a syringe, and a self-blood collection where a test subject collects blood in person by pricking a finger of his or her hand with a blood collecting needle.

Conventionally the blood collected by normal blood collection system is carried to a test station as contained in an airtight container, where the blood is tested after it has been separated into blood cell and blood plasma by a centrifugal separator. On the other hand, the blood collected by self-blood collection system is carried to the test station as dried after impregnated into a filter paper, where an analyses is conducted in a way that a white part of the filter paper representative of the blood plasma is cut off at the test station and then is dissolved into a solvent, leaving a red part thereof representative of the blood cell.

However, in the normal blood collection system, since the supernatant blood plasma has to be sucked by a dropping pipet and transferred into a special container prepared for blood plasma analyses after the collected blood has been centrifugally separated, the operation for separating the blood into the blood cell and the blood plasma has taken rather long time, leading to higher cost, and further the operation for transferring the blood plasma into the special container has a potential risk of mix-up or the similar accidents.

Still further, due to the fact that the blood cell in the blood is hemolyzing as time goes by and the accuracy guarantee period of the blood left at ambient temperature is not more than one day, in the case where the test has been conducted after that period, there occurs some problems that affect test results or measured values to be no more available as a polestar for medical care and diagonosing, including that the measured values of electrolytic materials, such as sodium, kalium, chrome and the likes, might be adversely effected in their accuracy and the numeric values of enzyme system, such as GOT, GPT and the likes could not be measured.

Yet further, to separate the blood by a centrifugal separator and to conduct a test on predetermined items, the blood amount of about 5 to 10 ml is required for each collection. Accordingly, since it is difficult for a test subject in person to collect the blood, but a specifically qualified person, such as a doctor, has to collect the blood, the person of test subject is required to go to the hospital or the likes, or otherwise the qualified person has to go to the place of the person of test subject, thus requiring a long time and much labor for blood collection.

On the other hand, in the self-blood collection, since a series of steps is required comprising impregnating the blood into a filter paper, drying the filter paper, and then dissolving the filter paper into a solvent, the self-blood collection is usable to the test exclusively on such item that the test value could not be affected by going through those steps, but is not employable for the test on the other items.

Accordingly, the present invention, in the light of the problems described above, provides an inventive instrument and method for separating blood, which accomplishes a reduction of cost in a blood test, a longer shelf life of the blood, and an improved test accuracy with a small amount of blood to be collected and a simplified operation.

The present invention is made in the light of the situations described above and has features below to solve the problems mentioned above. That is to say, a blood separating instrument according to the present invention comprises: a blood collection means for containing collected blood; a filtration means for separating blood cell and blood plasma in said collected blood; a blood cell collection means for containing separated blood cell; a blood plasma collection means for containing separated blood plasma; and a pressure applying means for applying pressure to the blood contained in said blood collection means; wherein said filtration means includes a capillary having one end in communication with a blood discharging section of said blood collection means and the other end in communication with a blood cell introducing section of said blood cell collection means, said capillary having a plurality of perforations formed through a wall thereof with a size to allow the blood plasma to pass therethrough but to prevent the blood cell from passing therethrough, so that when the blood in said blood collection means is introduced into said filtration means by a pressure applying motion caused by said pressure applying means, the blood plasma in said blood may move through said perforations of said filtration means to be separately contained in said blood plasma collection means.

In a preferred aspect of the present invention, a blood separating instrument comprises: a main container body having a blood collection container portion for containing collected blood, a blood plasma collection container attaching/detaching portion in communication with a blood discharging section of said blood collection container portion, and a blood cell collection container attaching/detaching portion whose blood cell introducing section is in communication with said blood plasma collection container attaching/detaching portion; a push-in cap having a push-in section which is adapted to be fittingly inserted into said blood collection container portion; a blood plasma collection container detachably connected with said blood plasma collection container attaching/detaching portion and containing a predetermined amount of blood plasma dilution; a blood cell collection container detachably connected with said blood cell collection container attaching/detaching portion and containing a predetermined amount of blood cell protective solvent; and an ultrafiltration element for separating the blood cell and the blood plasma in said blood; wherein said ultrafiltration element has a group of capillaries made of filter paper each having one end in communication with said blood discharging section of said blood collection container portion and the other end in communication with said blood cell introducing section of said blood cell collection container, said capillary having a plurality of perforations formed through a wall thereof with a size to allow the blood plasma to pass therethrough but to prevent the blood cell from passing therethrough, so that when the blood in said blood collection container portion is introduced into said ultrafiltration element by a pushing-in motion of said push-in cap, the blood plasma in said blood may move through said perforations of said ultrafiltration element to be separately contained in said blood plasma collection container.

Further, in a preferred aspect of the present invention, an inner diameter of the perforation of said capillary is within a range of 0.4 to 0.6 µ m, said blood collection container portion contains spherical solvent enclosed by a sheet wall arranged in a bottom portion thereof, said push-in section of said push-in cap has an end portion capable of collapsing said sheet wall, and an outer diameter of each element of said spherical solvent is greater than the inner diameter of the perforation of said capillary.

Still preferably, a volume of said blood collection container portion is within a range of 80 to 120 µliter, and said instrument of the present invention further comprises a piston which divides the inside of said blood cell collection container into an air layer region and a blood cell protective solvent containing region and is capable of slidably moving therein, wherein a pressure in said air layer region is set to be within a range of 1.0 to 1.5 atm., and said blood plasma dilution is mixed with a predetermined amount of pigment.

A blood separating method according to the present invention comprises the steps of: collecting blood into a blood collection means; applying a pressure to said blood by a pressure applying means to introduce said blood into a filtration means whose one end communicates with a blood discharging section of said blood collection means; containing blood plasma in said blood into a blood plasma collection means through perforations formed through a wall of said filtration means; and making blood cell in said blood flow through said filtration means to be contained in a blood cell collection means through a blood cell introducing section of said blood cell collection means, with which the other end of said filtration means communicates.

In a preferred aspect of the present invention, a blood separating method comprises the steps of; collecting blood into a blood collection container portion of a main container body; fittingly inserting and pushing-in a push-in cap into said blood collection container portion immediately after said collecting step to introduce said blood into a capillary of an unltrafiltration element whose one end communicates with a blood discharging section of said blood collection container portion; dissolving blood plasma in said blood through perforations formed through a wall of said capillary into blood plasma dilution contained in a blood plasma collection container connected air-tightly with said main container body,; and making blood cell in said blood flow through said ultrafiltration element to be mixed into blood cell protective solvent contained in a blood cell collection container connected air-tightly with said main container body through a blood cell introducing section of said blood cell collection container with which the other end of said ultrafiltration element communicates.

Further, in a preferred aspect of the present invention, said push-in section of said push-in cap is pushed-in to reach to the lowest level to collapse a sheet wall arranged in a bottom portion of said blood collection container for enclosing the spherical solvent and to fill up each of said capillaries with said spherical solvent to be coagulated therein, and further said blood is collected in an amount of 80 to 120 µliter.

According to the present invention described above, said blood flows from said blood collection means into the capillary of said filtration means, wherein the blood plasma of said blood passes through the perforations of said capillary to be contained in said blood plasma collection means, while the blood cell of said blood flows through said capillary to be contained in said blood cell collection means.
Fig. 1 is a cross sectional view of an embodiment of the present invention;
Fig. 2 is a cross sectional view of a main container body according to the present invention;
Fig. 3 is a top view of the main container body according to the present invention;
Fig. 4 is a bottom view of the main container body according to the present invention;
Fig. 5 is a schematic view of an ultrafiltration element of the present invention;
Fig. 6 is a partial perspective view of a capillary according to the present invention;
Fig. 7 is a cross sectional view of a blood plasma collection container according to the present invention;
Fig. 8 is a side elevational view of the blood plasma collection container according to the present invention;
Fig. 9 is a top view of the blood plasma collection container according to the present invention;
Fig. 10 is a bottom view of the blood plasma collection container according to the present invention;
Fig. 11 is a cross sectional view of a blood cell collection container according to the present invention;
Fig. 12 is a side elevational view of the blood cell collection container according to the present invention;
Fig. 13 is a top view of the blood cell collection container according to the present invention;
Fig. 14 is a bottom view of the blood cell collection container according to the present invention;
Fig. 15 is a cross sectional view of a push-in cap according to the present invention;
Fig. 16 is a side elevational view of the push-in cap according to the present invention;
Fig. 17 is a top view of the push-in cap according to the present invention;
Fig. 18 is a bottom view of the push-in cap according to the present invention;
Fig. 19 is a cross sectional view, illustrating the blood having been collected in the blood collection container portion, according to the present invention;
Fig. 20 is a cross sectional view, illustrating a push-in section of the push-in cap being pushed-in against the blood collection container portion, according to the present invention;
Fig. 21 is a cross sectional view, illustrating a sheet wall arranged in a bottom portion of the blood collection container portion being collapsed and a spherical solvent being discharged therefrom, according to the present invention;
Fig. 22 is a cross sectional view, illustrating the push-in cap having been pushed-in to the lowest level, according to the present invention;

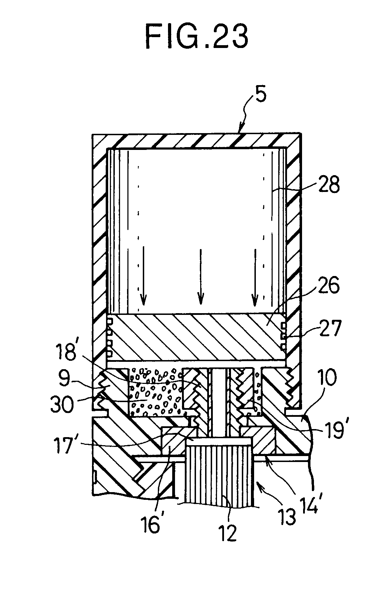

Fig. 23 is a cross sectional view, illustrating the blood cell collection container in its initial state, according to the present invention;
Fig. 24 is a cross sectional view, illustrating the blood cell starting to flow into the blood cell collection container, according to the present invention;
Fig. 25 is a cross sectional view, illustrating a piston being raised up temporarily, according to the present invention; and

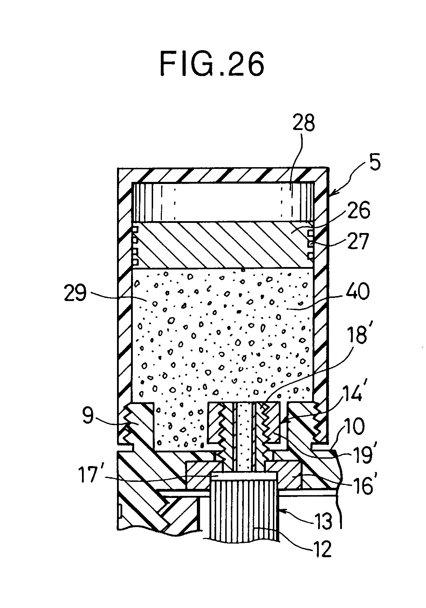

Fig. 26 is a cross sectional view, illustrating the inflow of the blood cell into the blood cell collection container having been completed, according to the present invention.

A preferred embodiment of the present invention will now be described with reference to the attached drawings.

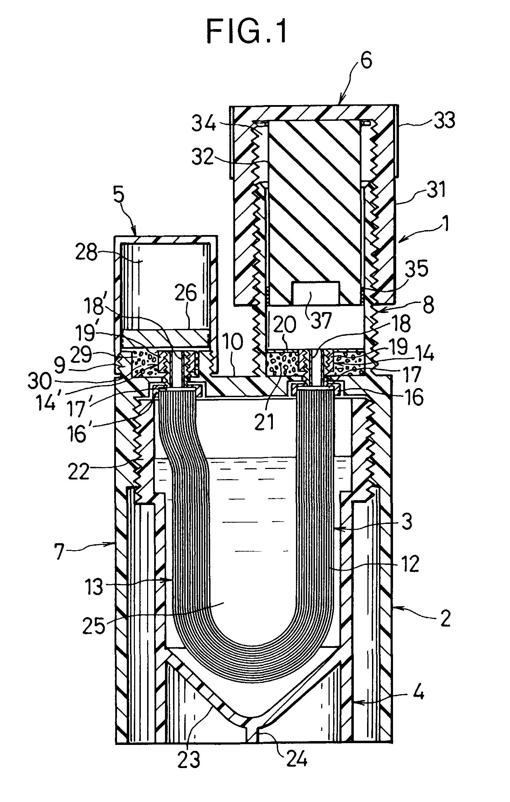

Fig. 1 shows a blood separating instrument 1 according to the present invention, which comprises: a main container body 2; an unltrafiltration element 3 connected with said main container body 2; a blood plasma collection container 4 and a blood cell collection container 5, both of which are respectively detachably connected with said main container body 2; and a push-in cap 6 adapted to be fittingly inserted into said main container body 2; wherein said main container body 2, said blood plasma collection container 4 and said blood cell collection container 5 are typically made of synthetic resin.

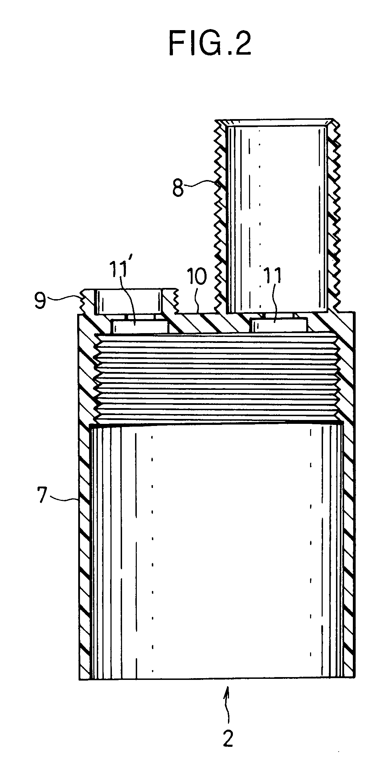

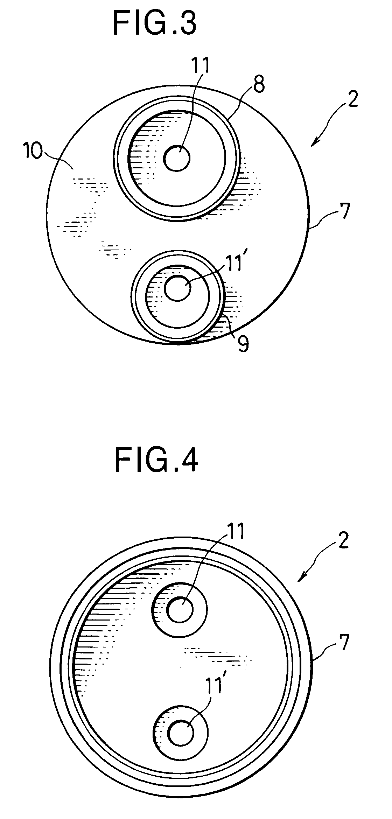

Referring to Figs. 2 to 4, said main container body 2 is formed into a cylindrical shape having a ceiling and comprises a blood plasma collection container attaching/detaching portion 7 having an inner diameter of, for example, about 1.5 cm, and a blood collection container portion 8 and a blood cell collection container attaching/detaching portion 9, each being formed into a cylindrical shape on said blood plasma collection container attaching/detaching portion 7 respectively, wherein said blood collection container portion 8 has a bore diameter and a height both being greater than those of said blood cell collection container attaching/detaching portion 9. Preferably, said blood collection container portion 8 has a volume within a range of 80 to 120 µliter (equivalent to 4 or 5 drops of blood), and more preferably a volume of 100 µliter, that is, for example, when the bore diameter is 8 mm, the depth is about 2mm. A blood discharging channel 11 and a blood cell introducing channel 11' are formed through a top plate 10 of said blood plasma collection container attaching/detaching portion 7, so that said blood collection container portion 8 communicates with said blood plasma collection container attaching/detaching portion 7 through said blood discharging channel 11, and said blood cell collection container attaching/detaching portion 9 communicates with said blood plasma collection container attaching/detaching portion 7 through said blood cell introducing channel 11'. Said blood plasma collection container attaching/ detaching portion 7 is threaded in the upper inner side thereof, while said blood collection container portion 8 and said blood cell collection container attaching/detaching portion 9 are respectively threaded in the outer sides thereof.

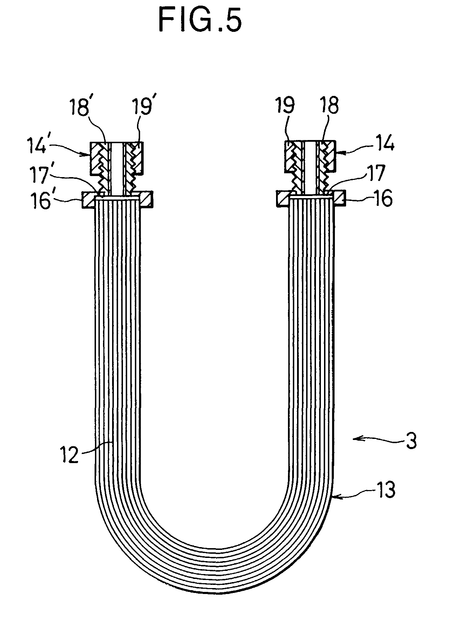

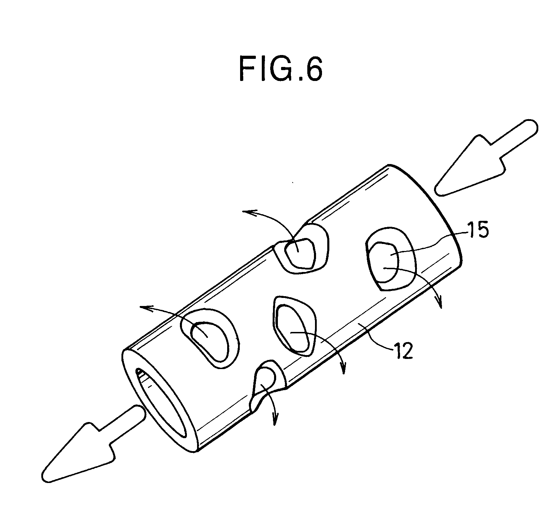

As shown in Figs. 1, 5 and 6, said ultrafiltration element 3 has a filtering section 13 comprising a plurality of capillaries 12, for example, 100 strings of capillaries made of filter paper (e.g. each of capillaries having the bore diameter of 2mm and the total length of 3 cm), and a connecting section 14 and 14' respectively arranged in either end portions of said filtering section 13, in which each of said capillaries 12 has a plurality of perforations 15 formed through a wall thereof, said perforation preferably having an inner diameter within a range of 0.4 to 0.6 µm and more preferably an inner diameter of 0.5 µm. Each of said connection sections 14 and 14' has a flat pedestal 16 or 16' for respectively binding said capillaries 12, and each of said pedestals 16 and 16' is adapted to form a confluent section 17 or 17' of flat space inside thereof as respectively binding said capillaries 12. Further, conduits 18 and 18' are respectively fixedly attached onto the upper central portions of ceilings of said pedestals 16 and 16' to communicate with said confluent sections 17 and 17', said conduits 18 and 18' being threaded on the outer surfaces respectively. Said conduits 18 and 18' are respectively arranged so as to pass through said blood discharging channel 11 and said blood cell introducing channel 11' from the blood plasma collection container attaching/ detaching portion 7 side respectively, wherein said conduit 18 is to be engaged with a nut 19 screwed from said blood collection container portion 8 side, while said conduit 18' is to be engaged with a nut 19' screwed from said blood cell collection container attaching/detaching portion 9 side, so that said nuts 19 and 19' and said pedestals 16 and 16' cooperate to clamp the top plate 10 of said blood plasma collection container attaching/detaching portion 7. Further, said blood collection container portion 8 has a sheet wall 20 made of material capable of being collapsed, such as synthetic rubber, horizontally extended across the bottom portion thereof, and said conduit 18 is arranged so as to air-tightly penetrate said sheet wall 20, wherein under said sheet wall 20 is contained a predetermined amount of artificial spherical solvent 21 composed of elements each having an outer diameter larger than the inner diameter of said perforation 15 of said capillary 12, preferably about 0.2 µm greater than the inner diameter of said perforation 15.

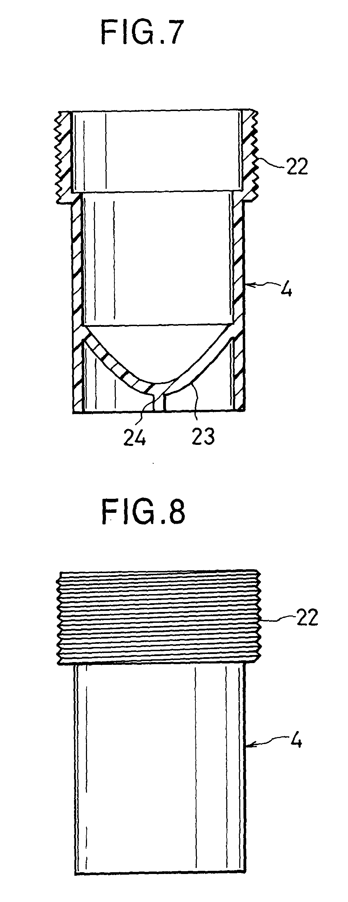

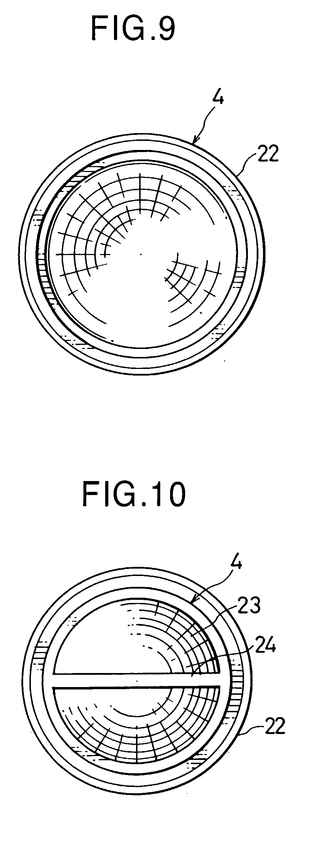

Referring to Fig. 1 and Figs. 7 to 10, said blood plasma collection container 4 is formed into an approximately cylindrical shape having an expanded diameter section 22 arranged in the upper portion thereof and a bottom section 23 arranged in the lower portion thereof, preferably such a shape that allows the container 4 to be directly set in a blood plasma analyzer (not shown). Said expanded diameter section 22 is threaded on the outer surface thereof so that the expanded diameter section 22 may be screwed into and engaged with said blood plasma collection container attaching/detaching portion 7. Said bottom section 23 is formed into cone shape having a pinch section 24 protruding on the bottom surface along a radial direction so that said blood plasma collection container 4 could be attached to/detached from said blood plasma collection container attaching/detaching portion 7 by horizontally rotating said pinch section 24. Still further, said blood plasma collection container 4 receives blood plasma dilution 25 contained therein and in addition, pigment of malachite green or the like mixed in said blood plasma dilution 25.

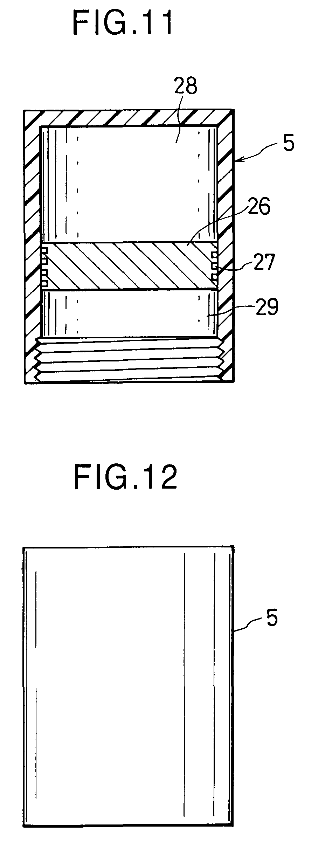

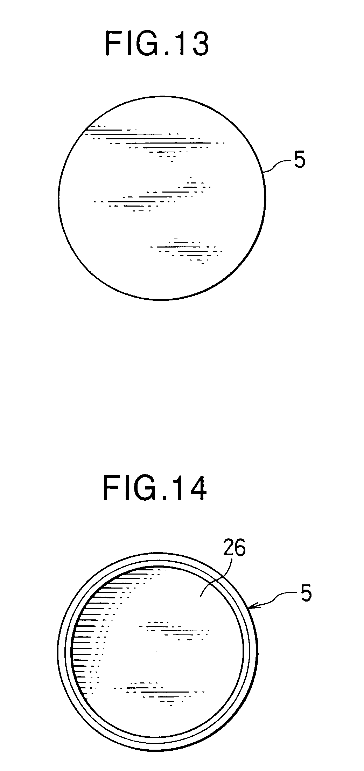

Referring to Fig. 1 and Figs. 11 to 14, said blood cell collection container 5 is formed into a cylindrical shape having ceiling, and is threaded on an inner lower portion thereof so that the container 5 is allowed to be attached to/detached from said blood cell collection container attaching/detaching portion 9. Further, a piston 26 formed into flat column or thick disk is arranged in said blood cell collection container 5 so as to be capable of sliding up and down therein. Preferably, a plurality of small grooves is carved on a peripheral wall of said piston 26 along a circular direction thereof, so that air tightness between the inner surface of said blood cell collection container 5 and said piston 26 may be retained while allowing said piston 26 to slide along said inner surface. The inside of said blood cell collection container 5 is divided by said piston 26 into two chambers, the chamber above said piston 26 defining an air layer region 28 while the other chamber beneath said piston 26 defining a blood cell protective solvent containing region 29. In order to prevent blood cell clotting and hemolysis, said blood cell protective solvent containing region 29 receives a blood cell protective solvent 30, for example, an anticoagulant such as EDTA or citric acid, contained therein, with the volume of about 1/7 of blood cell volume to be collected. Further, a pressure inside said air layer region 28 is kept in a level higher than the outside pressure, preferably 0.2 to 1.0 higher than the outside pressure so that said piston 26 may be in contact with said connecting section 14' in the initial state, preventing the backflow of said blood cell protective solvent 30 into said filtering section 13.

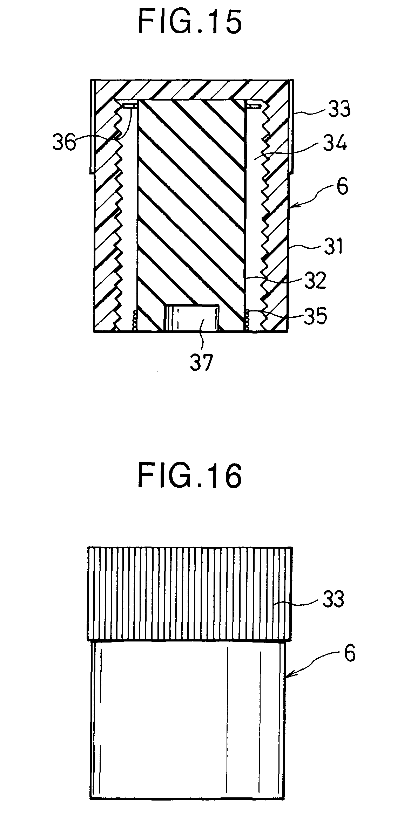

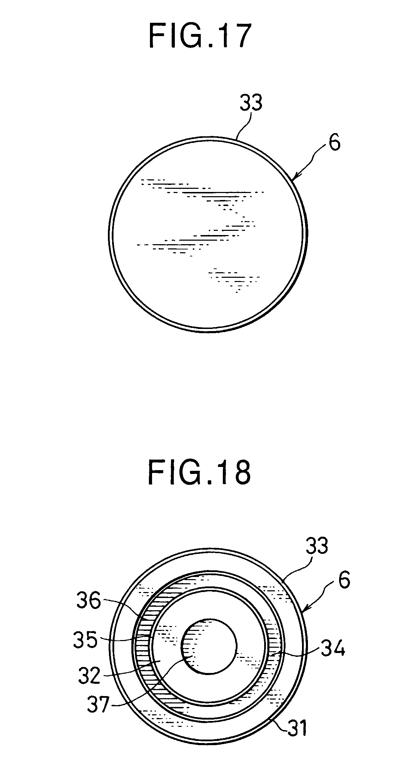

Referring to Fig. 1 and Figs. 15 to 18, said push-in cap 6 comprises an outer peripheral section 31 of cylindrical shape having a ceiling and made of synthetic resin, and a push-in section 32 of column shape made of synthetic rubber and fixedly connected to an inner surface of the top plate of said outer peripheral section 31 to be concentric therewith, wherein said outer peripheral section 31 is provided with a non-slip section 33 arranged on the upper outer surface thereof and is threaded on an inner peripheral surface thereof. Between said outer peripheral section 31 and said push-in section 32 is formed a space 34 of cylindrical shape, so that said outer peripheral section 31 could be screwed over said blood collection container portion 8 and thereby said push-in section 32 could be guided to be fittingly inserted into said blood collection container portion 8. On a lower portion of the peripheral wall of said push-in section 32 is arranged a sealing member, for example, a plurality of rings 35 made of resilient material, for retaining the air-tightness between said push-in section 32 and said blood collection container portion 8 while the former being fittingly inserted into the latter. Further, a packing 36 in the form of annular sheet plate is fixedly attached to a top portion of the peripheral wall of said push-in section 32, so that the air-tightness between said blood collection container portion 8 and said push-in cap 6 is retained by pressing the top end of said blood collection container portion 8 against said packing 36. Still further, a concave portion 37 is concentrically formed on the lower end of said push-in section 32, so that said concave portion 37 may be fittingly engaged with said nut 19.

A blood separating method according to the present invention will now be described with reference to the attached drawings.

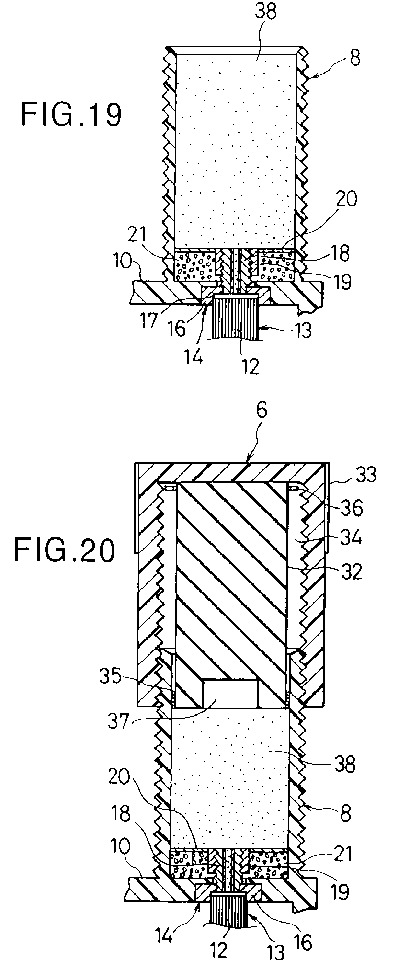

A person of test subject pricks a finger of his or her hand with a blood collecting needle to collect preferably 80 to 120 µliter (4 or 5 drops), more preferably 100 µliter of blood 38 into said blood collection container portion 8 as shown in Fig. 19. The person grasps said non-slip section 33, and fittingly inserts said push-in section 32 of said push-in cap 6 into said blood collection container portion 8 as shown in Fig. 20. The air tightness between said push-in section 32 and said blood collection container portion 8 is retained by said ring 35, said blood 38 is pressed by said push-in section 32 to flow through said conduit 18 into said respective capillaries 12 of said ultrafiltration element 3.

Since the blood plasma 39 in said blood 38 has an outer diameter smaller than the inner diameter of said perforation 15 of said capillary 12, therefore the blood plasma 39 goes through the perforation 15 to be dissolved into said blood plasma dilution 25 contained in said blood plasma collection container 4. With the help of the pigment mixed in the blood plasma dilution 25, a dilution ratio (dissolution ratio) of the solution could be accurately calculated by measuring a amount of the pigment per unit volume thus to keep the higher level of inspection accuracy of the blood plasma.

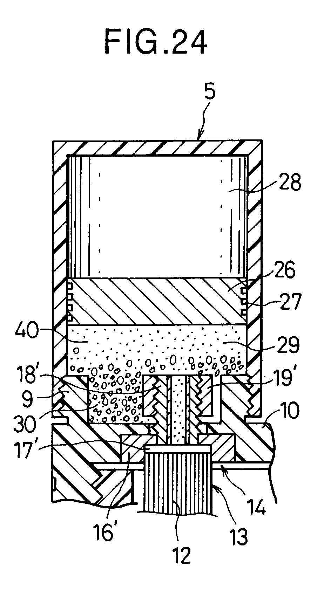

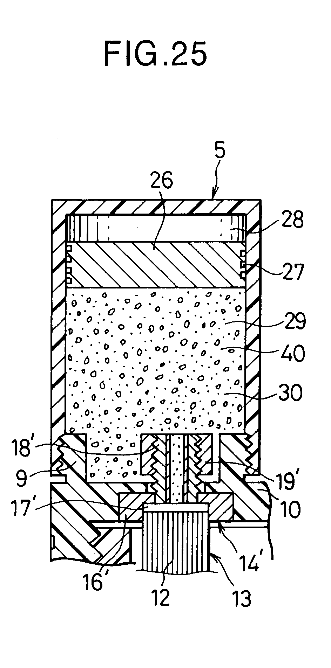

Further, since the blood cell 40 in said blood 38 has an outer diameter greater than the inner diameter of said perforation 15, the blood cell 40 flows through said respective capillaries 12 and said conduit 18' to raise the piston 26 as shown in Fig. 24, and to be finally mixed into said blood cell protective solvent 30 in said blood cell collection container 5. Since said grooves 27 are arranged on the peripheral wall of said piston 26, said piston 26 slidably moves upward while retaining the air-tightness between the inner peripheral surface of said blood cell collection container 5 and the piston 26. At that time, since it takes a certain period of time for said blood plasma 39 to pass through said perforation 15, said piston 26 is raised up temporarily as shown in Fig. 25 when said push-in section 32 is inserted at a burst, but since the pressure in said air region 28 is increased as the piston 29 is raised up, the increased pressure is then applied to said piston 26 to be slid down and to press said blood cell protective solvent 30 including said blood cell 40 mixed therein, thus accelerating said blood plasma 39 to pass through said perforation 15.

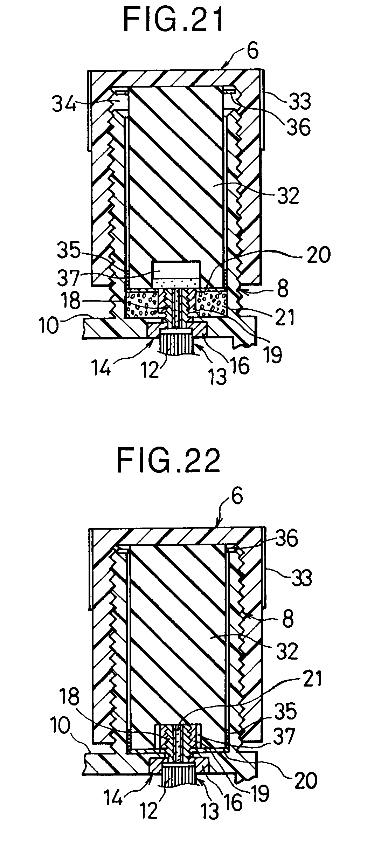

When said blood 38 in said blood collection container portion 8 has been completely discharged from said blood collection container portion 8, the lower end of said push-in section 32 collapses said sheet wall 20, and said spherical solvent 21 flows into said concave portion 37 of said push-in section 32 and pass though said conduit 18 into said respective capillaries 12, as shown in Figs. 21 and 22. When the push-in section 32 of said push-in cap 6 has reached to the lowest level, all of said spherical solvent 21 has flown out of said blood collection container portion 8 to fill up said respective capillaries 12 to be coagulated therein.

Accordingly, after that, said blood plasma dilution 25 including said blood plasma 39 dissolved therein is prevented from backflowing from said blood plasma collection container 4 to said respective capillaries 12, and said blood cell protective solvent 30 including said blood cell 40 mixed therein is also prevented from backflowing from said blood cell collection container 5 to said respective capillaries 12. Further, when said push-in section 32 has been pushed down and has reached to the lowest level, the upper end of said blood collection container portion 8 presses said packing 36, so that the air-tightness between said push-in cap 6 and said blood collection container portion 8 could be doubly secured by said packing 36 in addition to the effect of said ring 35.

Subsequently, said blood separating instrument 1, having said blood 38 as separated into said blood plasma 39 and said blood cell 40, is carried to a test station for the test on determined items to be conducted.

In this case, since the blood has been separated into the blood plasma and the blood cell right on the spot immediately after the blood collection and then the blood is carried to the test station with the blood cell included in the blood cell protective solvent, therefore hemolysis, blood clotting and the likes in transit could be prevented. Accordingly, the shelf life of the blood could be improved, thus to enhance the test accuracy. Further, since the collected blood is conservable for about one week at ambient temperature, any arrangement, such as quick transportation or geographical consideration of a collection spot and a test station, is not necessary, thus to improve a degree of flexibility in operation. Still further, the blood separation could be done with only a few drops of collected blood because no centrifugal separator is used, and thus the self-blood collection is usable for the test on the items equivalent to those tested by employing a conventional normal blood collection.

It should be appreciated that, although in the embodiment described above, said blood plasma collection container 4, said blood cell collection container 5, and said push-in cap 6 are threaded respectively to be engaged with said main container body 2, any alternative connection method may be applicable so far as it is detachable and allows the air-tightness to be retained, including a tapering without threading.

Further, to prevent a damage to blood cell by a projection within the containers, the inner surfaces of the containers including said blood cell collection container 5, said blood collection container portion 8 or the likes may be coated with heparin or the likes.

Still further, it is needless to say that the materials of said main container body 2, said blood plasma collection container 4, said blood cell collection container 5, said push-in cap 6, said piston 26 and the likes are not limited to those described above.

Yet further, although in the embodiment described above, the description has been made on the case where the self-blood collection is employed, it is needless to say that the present invention is applicable to the case where the normal blood collection is employed.

According to the present invention described above, since a separating process of blood into the blood plasma and the blood cell could be done only by applying the pressure to the collected blood, a variety of effects is advantageously provided, including that the reduction in operational cost as well as the simplification in operation could be accomplished.

## Claims

1. A blood separating instrument comprising:
a blood collection means for containing collected blood;
a filtration means for separating blood cell and blood plasma in said collected blood;
a blood cell collection means for containing separated blood cell;
a blood plasma collection means for containing separated blood plasma; and
a pressure applying means for applying pressure to the blood contained in said blood collection means;
wherein, said filtration means includes a capillary having one end in communication with a blood discharging section of said blood collection means and the other end in communication with a blood cell introducing section of said blood cell collection means, said capillary having a plurality of perforations formed through a wall thereof, each of said perforation having a size to allow the blood plasma to pass therethrough but to prevent the blood cell from passing therethrough;
said instrument characterized in that the blood in said blood collection means is introduced into said filtration means by a pressure applying motion caused by said pressure applying means, and the blood plasma in said blood moves through said perforations in said filtration means to be separately contained in said blood plasma collection means.

2. A blood separating instrument comprising:
a main container body having a blood collection container portion for containing collected blood, a blood plasma collection container attaching/detaching portion in communication with a blood discharging section of said blood collection container portion, and a blood cell collection container attaching/detaching portion whose blood cell introducing section is in communication with said blood plasma collection container attaching/detaching portion;
a push-in cap having a push-in section which is adapted to be fittingly inserted into said blood collection container portion;
a blood plasma collection container detachably connected with said blood plasma collection container attaching/detaching portion and containing a predetermined amount of blood plasma dilution;
a blood cell collection container detachably connected with said blood cell collection container attaching/detaching portion and containing a predetermined amount of blood cell protective solvent; and
an ultrafiltration element for separating the blood cell and the blood plasma in said blood;
wherein said ultrafiltration element has a group of capillaries made of filter paper each having one end in communication with said blood discharging section of said blood collection container portion and the other end in communication with said blood cell introducing section of said blood cell collection container, said capillary having a plurality of perforations formed through a wall thereof, each of said perforation having a size to allow the blood plasma to pass therethrough but to prevent the blood cell from passing therethrough;
said instrument characterized in that the blood in said blood collection container portion is introduced into said ultrafiltration element by a pushing-in motion of said push-in cap, and the blood plasma in said blood moves through said perforations of said ultrafiltration element to be separately contained in said blood plasma collection container.

3. A blood separating instrument in accordance with claim 2, in which an inner diameter of said perforation of the capillary is within a range of 0.4 to 0.6 µm.

4. A blood separating instrument in accordance with either of claim 2 or 3, in which said blood collection container portion contains spherical solvent enclosed by a sheet wall arranged in a bottom portion thereof, and said push-in section of said push-in cap has an end portion capable of collapsing said sheet wall.

5. A blood separating instrument in accordance with claim 4, in which an outer diameter of each element of said spherical solvent is greater than the inner diameter of said perforation of said capillary.

6. A blood separating instrument in accordance with either of claim 2 to 5, in which a volume of said blood collection container portion is within a range of 80 to 120 µliter.

7. A blood separating instrument in accordance with either of claim 2 to 6, further comprising a piston which divides the inside of said blood cell collection container into an air layer region and a blood cell protective solvent containing region, and is capable of slidably moving therein.

8. A blood separating instrument in accordance with claim 7, in which a pressure in said air layer region is set to be a level 0.2 to 1.0 atm. higher than an outside pressure.

9. A blood separating instrument in accordance with claim 2, in which said blood plasma dilution is mixed with a predetermined amount of pigment.

10. Ablood separating method comprising the steps of:
collecting blood into a blood collection means;
applying a pressure to said blood by a pressure applying means to introduce said blood into a filtration means whose one end communicates with a blood discharging section of said blood collection means;
containing blood plasma in said blood into a blood plasma collection means through perforations formed through a wall of said filtration means; and
making blood cell in said blood flow through said filtration means to be contained into a blood cell collection means through a blood cell introducing section of said blood cell collection means with which the other end of said ultrafiltration element communicates.

11. A blood separating method comprising the steps of:
collecting blood into a blood collection container portion of a main container body;
fittingly inserting and pushing-in a push-in cap into said blood collection container portion immediately after said collecting step to introduce said blood into a capillary of an ultrafiltration element whose one end communicates with a blood discharging section of said blood collection container portion;
dissolving blood plasma in said blood through perforations formed through a wall of said capillary into blood plasma dilution contained in a blood plasma collection container connected air-tightly with said main container body; and
making blood cell in said blood flow through said ultrafiltration element to be mixed into blood cell protective solvent contained in a blood cell collection container connected air-tightly with said main container body through a blood cell introducing section of said blood cell collection container with which the other end of said ultrafiltration element communicates.

12. A blood separating method in accordance with claim 11, further comprising the steps of:
pushing-in said push-in section of said push-in cap to the lowest level;
collapsing a sheet wall arranged in a bottom portion of said blood collection container for enclosing a spherical solvent, and
filling up each of said capillaries with said spherical solvent to be coagulated therein.

13. A blood separating method in accordance with either of claim 11 or 12, in which said blood is collected in an amount of 80 to 120 µliter.
